# FASCICULE DE BREVET EUROPEEN

(11) **EP 2 804 182 B1**
(45) Date de publication et mention de la délivrance du brevet: **23.03.2016**
(21) Numéro de dépôt: 14002681.6
(22) Date de dépôt: 24.03.2011
(51) Int. Cl.: G21F 5/018, A61M 5/178, B65B 3/00

(54) **Fourreau de protection et de préhension d'une seringue**
Hülse zum Schützen und Halten einer Injektionsspritze
Sheath for protection and gripping of a syringe

(30) Priorité: 07.04.2010 FR 1001428
(43) Date de publication de la demande: 19.11.2014
(62) Demande divisionnaire de: 11730339.6
(73) Titulaire: ISP System, 65500 Vic-en-Bigorre Cedex (FR)
(72) Inventeur: Sauvageot, Paul, 65500 Camales (FR); Brescon, David, 65320 Gayan (FR)
(74) Mandataire: Célanie, Christian

(56) Documents cités:
- EP-A1- 1 317 299
- JP-A- 2005 021 247
- US-A- 3 814 941
- US-A- 3 973 554
- US-A1- 2007 219 505

## Description

Le secteur technique de la présente invention est celui des injecteurs de produits radioactifs utilisés dans l'imagerie par tomographie d'émission de positons et la radiothérapie interne vectorisée.

Pour la scintigraphie, un produit radioactif est injecté au patient, le produit injecté produisant un rayonnement mesuré ensuite par des techniques d'imagerie. Il est très important de protéger au maximum les opérateurs manipulant les produits radioactifs. Les opérateurs sont en effet soumis à des risques d'expositions répétées aux rayonnements ionisants. L'injecteur est par exemple un équipement robotisé qui doit permettre d'apporter cette protection aux opérateurs lors des trois phases principales de manipulation : la préparation de la dose pour le patient, le transport de cette dose du laboratoire de radiopharmacie jusque dans la salle d'injection et enfin l'injection de la dose au patient. Le dispositif de préparation d'une seringue est également désigné par dispositif de pipetage.

Le fluide radioactif est par exemple stocké dans des conteneurs blindés avant d'être aspiré dans un réservoir de seringue. Les seringues servant pour l'injection de produit radioactif sont par exemple disposées dans des dispositifs blindés. Le document EP-1317299 décrit un exemple de dispositif de protection pour seringue servant pour l'injection de produit radioactif. Le document FR-2739565 décrit un exemple de procédé et un exemple d'installation pour la préparation d'une solution radioactive injectable.

Le but de la présente invention est d'améliorer les dispositifs existants notamment en proposant une structure plus pratique.

L'invention a donc pour objet un un fourreau de protection et de préhension d'une seringue équipée d'une aiguille destiné à une mise en oeuvre dans le dispositif de préparation selon l'invention, fourreau comprenant d'une part un blindage tubulaire fixé autour d'un réservoir de la seringue, et d'autre part un fond, caractérisé en ce que ledit fourreau comprend deux tourillons support fixes par rapport à un blindage tubulaire, lesdits tourillons délimitant une surface cylindrique d'appui dans des logements du berceau, le berceau délimitant un réceptacle de réception dudit fourreau et un élément de blocage du fond par rapport au berceau, un élément de rappel tendant à entraîner l'élément de blocage dans une position de blocage en translation du fond par rapport au berceau.

Avantageusement, le fourreau comprend une enveloppe de guidage disposée autour du blindage tubulaire, ladite enveloppe étant équipée de fentes dans lesquelles circulent les tourillons, ladite enveloppe étant reliée au fond équipé d'un moyen d'attache à la tête du piston de la seringue. Les tourillons peuvent également être désignés par axes de préhension.

Avantageusement encore, le fourreau comprend un crochet mobile en rotation par rapport au blindage tubulaire, le crochet réalisant un accrochage automatique d'une collerette du réservoir de la seringue, un bouton poussoir étant disposé à une extrémité libre d'un des deux tourillons, le bouton poussoir agissant sur le crochet, à l'opposé d'un élément de rappel, pour dégager la collerette du crochet, le crochet étant escamoté par rapport au fourreau dans sa position d'accrochage.

Avantageusement encore, le moyen d'attache comprend deux arceaux articulés et rapprochés automatiquement l'un vers l'autre par un élément de rappel, les arceaux étant équipés chacun d'une oreille saillante disposée vers l'intérieur du fourreau et destinée à venir contre une face de ladite tête.

L'invention vise également le dispositif de transport ou d'injection d'une dose de fluide radioactif à injecter à un patient comprenant le fourreau.

On notera que le bâti se présente sous la forme d'une structure porteuse qui est en liaison avec les différents postes réalisant des opérations ou avec des organes fonctionnels, ces liaisons étant par exemple des liaisons de type pivot ou des fixations ou des liaisons de type glissière ou d'autres types de liaisons mécaniques. L'enceinte est par exemple blindée contre les rayonnements ionisants. L'enceinte comprend par exemple un espace intérieur dans lequel sont par exemple réalisées des manipulations, également désignées par opérations.

Le bâti est en liaison avec un organe de maintien d'un flacon, inclinable par rapport au bâti, le flacon formant la réserve de fluide radioactif et étant fermé par un couvercle transperçable, le flacon étant positionné automatiquement dans au moins une position verticale de perçage par l'aiguille de la seringue et une position d'aspiration inclinée d'un angle déterminé par rapport à la verticale.

Selon un avantage particulier, le dispositif de préparation comprend une aiguille d'appel d'air en liaison avec le bâti, ayant une inclinaison correspondant à ladite position inclinée du flacon, l'aiguille d'appel d'air étant mobile par rapport au bâti et commandée automatiquement au moins en translation selon son axe central, dans au moins une position de perçage du flacon incliné.

Selon un avantage de l'invention, le capteur produit au moins :
- des données correspondant à une augmentation déterminée de l'effort en translation verticale et représentatives d'un début d'aspiration de fluide radioactif dans la seringue ou
- des données correspondant à un effort en translation verticale nul et représentatives d'une libération du fond par la pince.

Ainsi, avec le dispositif selon l'invention toutes les opérations de remplissage de la seringue, d'enlèvement de l'aiguille de prélèvement de la dose de produit radioactif et de retour en position initiale du fourreau peuvent être réalisées de manière automatique sans intervention humaine.

Le dispositif selon l'invention permet avantageusement des interventions manuelles en cas de panne ou d'intervention volontaire d'un opérateur.

Le dispositif selon l'invention permet d'éviter toute contamination de l'opérateur préparant la dose et le fourreau permet le transport et l'injection au patient de la dose dans des conditions de sécurité élevées.

D'autres caractéristiques, avantages et détails de l'invention seront mieux compris à la lecture du complément de description qui va suivre de modes de réalisation donnés à titre d'exemple en relation avec des dessins sur lesquels :
- les figures 1 et 2 représentent chacune une vue en perspective d'un exemple de dispositif de préparation d'une seringue,
- la figure 3 représente une vue en perspective d'un exemple de bâti en liaison avec un poste de remplissage, un poste de l'enlèvement de l'aiguille et un organe de transfert, dans une position déterminée,
- la figure 4 représente une vue en perspective d'un exemple de seringue équipée d'une aiguille et disposée dans son fourreau,
- la figure 5 représente une vue en coupe de la seringue,
- la figure 6 représente une vue en perspective du piston de la seringue maintenu par des arceaux,
- la figure 7 représente un exemple de réalisation des arceaux,
- la figure 8 représente une vue en perspective d'un exemple de berceau supportant la seringue insérée dans son fourreau,
- la figure 9 représente une vue de dessus du berceau de la figure 8,
- la figure 10 représente une vue d'ensemble du dispositif selon l'invention,
- la figure 11 représente une vue d'un détail du bâti,
- la figure 12 représente une vue en perspective du bâti,
- les figures 13, 14 et 15 représentent des détails de réalisation du poste de prélèvement montrant un exemple de fourchette et de pince du poste de remplissage,
- les figures 16 et 17 représentent chacune une vue en perspective d'un exemple d'élément de blocage bloquant la partie de guidage du fourreau par rapport au berceau,
- les figures 18, 19, 20 et 21 représentent des vues en perspective d'un exemple de bâti en liaison avec un poste de remplissage, un poste de l'enlèvement de l'aiguille et un organe de transfert, dans des positions déterminées,
   les figures 22, 23 et 24 représentent des vues en perspective d'un exemple de flacon dans des positions déterminées par rapport au bâti,
- la figure 25 est une coupe d'un flacon de produit radioactif montrant le positionnement de l'aiguille,
- la figure 26 représente une vue en perspective d'un exemple d'organe de maintien d'un flacon comprenant le fluide de remplissage,
- les figures 27, 28 et 29 représentent le poste d'enlèvement de l'aiguille, et
- la figure 30 représente de façon schématique un exemple d'outil escamotable de dévissage du poste d'enlèvement de l'aiguille.

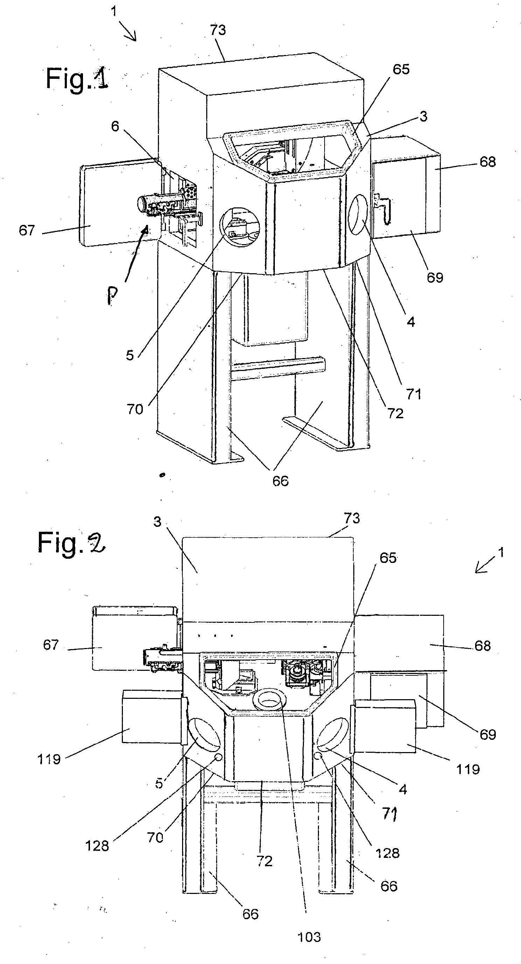

L'invention va à présent être décrite et sur les figures 1 et 2 on a représenté un dispositif de préparation 1 d'une seringue non visible sur ces figures comprenant une enceinte blindée 3 opaque aux émissions du produit radioactif contenu dans la seringue.

La figure 1 montre une vue de profil faisant apparaître les contours de l'enceinte 3 renfermant un volume intérieur protégé. La figure 2 est une vue de face plongeante montrant, de manière non limitative, un puits 103 de mesure de l'activité radioactive, installé à l'intérieur de l'enceinte 3. L'enceinte 3 est par exemple posée sur des pieds 66 reposant sur le sol.

L'enceinte 3 comprend par exemple une ouverture 6 latérale pouvant être fermée ou ouverte par une porte blindée 67 par laquelle on peut mettre en place la seringue à remplir ou la récupérer remplie de produit radioactif comme cela expliqué ci-après. Un capteur peut être prévu pour détecter par exemple la position verrouillée ou non de cette porte blindée 67 et transmettre un signal représentatif de cet état à un module électronique de gestion non représenté. Un boîtier 68 formant un sas d'accès permettant l'insertion de différents produits à l'intérieur de l'enceinte, est disposé sur un côté de l'enceinte, ce boîtier comportant par exemple une porte d'accès 69. L'enceinte comprend par exemple aussi une alimentation ou des portes d'accès à des actionneurs.

L'enceinte délimite un espace intérieur en deux volumes dont un premier est prolongé par un deuxième volume de hauteur moindre et dont le dessus est délimité par la fenêtre 65. Le deuxième volume en vue de dessus est délimité par deux bords, référencés 70 et 71, inclinés par rapport à un troisième bord 72 qui est parallèle au bord arrière 73 du premier volume.

L'enceinte 3 est munie de trous 4 et 5 à travers lesquels l'opérateur passe ses mains, réalisés dans les deux bords référencés 70 et 71. La forme de l'enceinte n'est pas limitative, et on pourrait envisager d'autres formes d'enceintes.

Grâce aux postes disposés à l'intérieur de l'enceinte, l'opérateur peut par exemple manipuler la seringue de façon semi-automatique, certaines opérations étant réalisées automatiquement. Des opérations automatiques sont réalisées avec toutes les portes blindées fermées, une condition de fonctionnement testée par le module électronique de gestion étant par exemple la réception par celui-ci des signaux représentatifs du verrouillage de chacune des portes blindées.

Le dispositif 1 de préparation permet notamment de préparer à l'aide de différents postes, une seringue équipée d'une aiguille et disposée dans un fourreau F de protection et de préhension.

L'opérateur commande ou réalise différentes opérations à travers une fenêtre 65 de visualisation de l'intérieur de l'enceinte 3 blindée et de trous 4 et 5 disposés sur le devant de l'enceinte 3 et permettant l'insertion des mains de l'opérateur. Ses mains sont par exemple protégées par des gants en latex qui évitent un contact éventuel avec du fluide radioactif. Les trous sont fermés ou ouverts par exemple par des portes blindées 119 articulées sur l'enceinte blindée 3. Des capteurs 128 permettent par exemple de transmettre au module électronique de gestion un signal représentatif de l'état verrouillé ou non de chacune de ces portes. Les parois de l'enceinte 3 ainsi que la fenêtre 65 ou les portes blindées sont par exemple isolantes contre les rayonnements ionisants.

Dans l'enceinte 3, on a prévu un bâti 2, auquel sont par exemple liés un élément de maintien du fourreau de la seringue et des postes de réalisation d'opérations que l'on va décrire plus complètement ci-après.

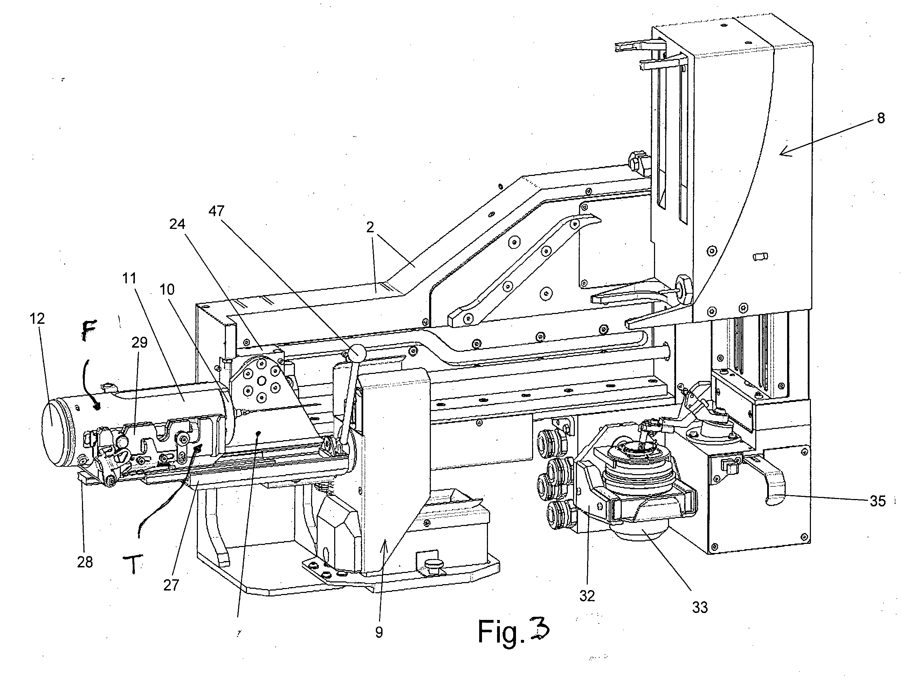

Sur la figure 3, on a représenté un exemple de réalisation d'un bâti 2 intégré dans le dispositif de préparation 1, sans l'enceinte blindée 3, devant lequel un opérateur peut se positionner pour réaliser ou commander les différentes phases.

Ce bâti 2 est relié à un poste de remplissage 8, un poste 32 de stockage de la réserve de produits radioactif, un organe T de transfert et de maintien de la seringue. De manière non limitative, les postes de réalisation des opérations peuvent être fixes ou mobiles par rapport au bâti 2. La seringue est par exemple transférée vide depuis sa position initiale de dépose P (figure 1) jusqu'au poste de remplissage puis passe à un poste 9 d'enlèvement de l'aiguille 7 avant son retour en position initiale. Le poste de remplissage 8 et le poste d'enlèvement 9 sont en liaison avec le bâti 2.

L'opérateur peut, à travers les trous 4 et 5 de l'enceinte, actionner un levier de commande 47 du poste 9 d'enlèvement de l'aiguille ou une mannette 35 commandant une rotation d'un socle portant un support de l'aiguille d'appel d'air, de façon à pouvoir positionner l'aiguille dans au moins une position fonctionnelle et une position de dégagement. L'opérateur dépose au départ la seringue S protégée par son fourreau F au poste de dépose P et la récupère après remplissage à ce poste.

Des actionneurs commandés par des organes électroniques de puissance ou des capteurs fournissant des signaux électriques représentatifs de leur état sont montés sur le dispositif de préparation. Des capteurs de fin de course sont installés dans le dispositif de préparation. Les opérations manuelles peuvent être contrôlées et validées par un ou plusieurs capteurs.

De manière non limitative, un module électronique de gestion, alimenté en énergie électrique, communique par des composants électroniques d'interface de communication et par des liaisons électriques, avec les actionneurs ou des capteurs. Le module électronique de gestion comprend par exemple un calculateur et un composant électronique de mémorisation comprenant un espace de mémorisation de données et un espace de mémorisation de programmes. Un port électronique de communication de type homme-machine permet par exemple à un opérateur d'interagir avec les programmes de traitement ou de stockage des données afin par exemple de commander les actionneurs, via une interface homme-machine reliée au module de gestion.

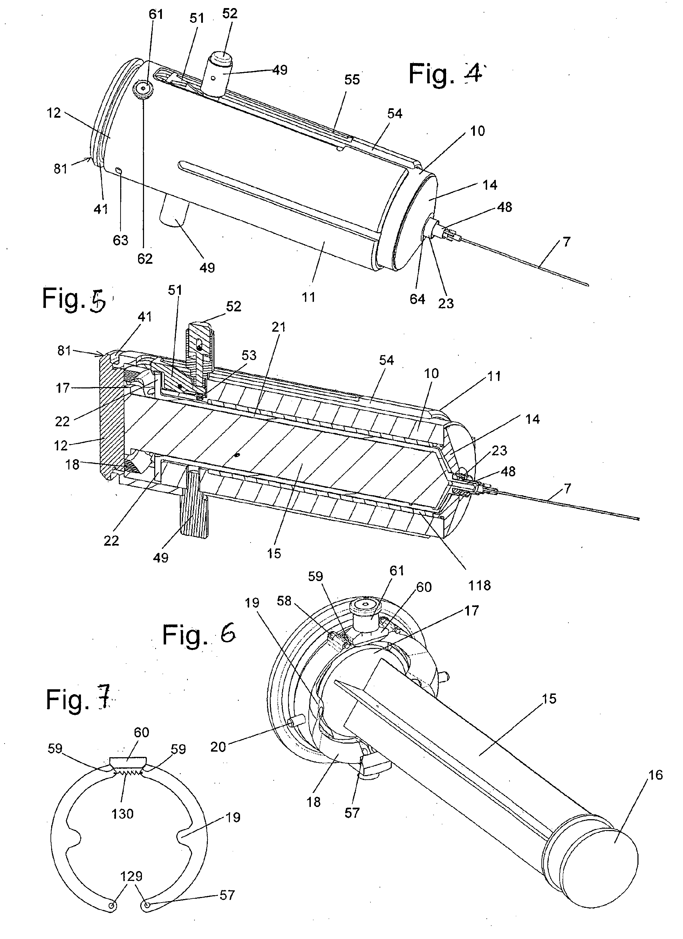

On va maintenant décrire le fourreau F de protection de la seringue S ainsi que le réceptacle du fourreau en relation avec les figures 4 à 9.

La figure 4 représente une vue en perspective du fourreau F qui comprend un blindage tubulaire 10 disposé autour d'un réservoir 21 de la seringue et la figure 5 une coupe longitudinale de ce fourreau.

Sur ces deux figures, le fourreau comprend un crochet 51 mobile en rotation par rapport au blindage tubulaire 10, le crochet 51 réalisant un accrochage automatique d'une collerette 22 du réservoir 21. Un bouton poussoir 52 est par exemple disposé à une extrémité d'un des deux axes 49 et agit sur le crochet 51, en s'opposant à l'action d'un ressort de rappel 53, dégageant ainsi la collerette 22 du crochet. Le crochet 51 est escamoté par rapport au fourreau dans sa position d'accrochage. La position escamotée permet de faciliter le positionnement du fourreau dans le berceau 29.

Le fourreau comprend une partie de guidage 11 disposée autour du blindage tubulaire 10, cette partie de guidage 11 étant équipée de fentes 54 par lesquelles passent les axes 49. Une de ces fentes 54, dans laquelle est escamoté le crochet 51, comprend des rainures 55 de limitation de course contre lesquelles viennent en appui des ailettes 56 du crochet 51.

La partie de guidage est liée à un fond 12 équipé d'une attache automatique à une tête 17 d'un piston 15 de la seringue. L'attache automatique à la tête 17 de piston 15 de la seringue comprend deux arceaux 18.

Une cloison intermédiaire 118 tubulaire favorisant le glissement peut être disposée entre le blindage tubulaire et le réservoir 21.

Le réservoir 21 de la seringue est rempli par un fluide radioactif.

Le fourreau comprend un capot blindé 14 ayant une ouverture centrale 64 par laquelle passe une base 48 de l'aiguille. La base 48 de l'aiguille 7 est vissée dans un logement de réception 23 fileté disposé à l'extrémité avant du réservoir 21.

Deux axes 49, fixes par rapport au blindage tubulaire 10, comprennent par exemple une surface cylindrique lisse d'appui dans des logements 50 du réceptacle. Ces axes disposés à l'opposé l'un de l'autre par rapport au blindage tubulaire 10, sont par exemple coaxiaux ou disposés avec un décalage déterminé entre leur axe géométrique.

Les axes 49 sont configurés de façon à maintenir le fourreau dans le réceptacle lorsqu'un effort de dévissage est exercé sur la base 48 de l'aiguille 7 et de façon à supporter la masse de la seringue et du fourreau disposés dans le réceptacle lorsque le réceptacle est en position verticale de remplissage.

Les parois cylindriques des axes 49 peuvent être coaxiales ou décalées de façon à réaliser un effet détrompeur pour le positionnement des axes dans leur logement 50.

Le berceau 29 comprend deux paires de logements 50 dont une paire de logements alignés et une paire de logements disposés l'un en face de l'autre mais légèrement décalés pour réaliser la fonction détrompeur. Chacune de ces paires de logements servira par exemple en fonction d'un type déterminé de fourreau ou de seringue.

Les axes 49 de préhension sont fixés au blindage tubulaire 10 par collage, par une goupille, par un ergot inséré dans un logement du blindage tubulaire 10 ou par tout autre moyen de fixation.

Les axes géométriques autour desquels sont centrés les axes 49 de préhension sont situés vers l'arrière par rapport au centre de gravité de la seringue dans son fourreau, que le réservoir 21 de la seringue soit rempli ou non.

Chaque arceau 18 comprend un plot 20 saillant vers l'extérieur du fourreau, ces plots 20 étant guidés dans des logements 63 réalisés dans le fond 12. Ces logements 63 ont une section transversale oblongue de largeur correspondant aux plots 20.

Le fond 12 comprend une rainure périphérique 41 et se termine par un bord 81 biseauté. Le fond 12 est disposé sur l'axe longitudinal de la seringue, derrière la tête 17 du piston 15. La tête de piston est réalisée sous la forme d'un plateau circulaire. Le piston 16 comprend un joint 16 à son extrémité opposée à la tête 17.

De manière non limitative, le fourreau comprend une fenêtre 109 permettant de visualiser le niveau de remplissage de la seringue. La fenêtre 109 est par exemple réalisée en verre blindé.

Le blindage tubulaire 10 et le capot blindé 14 sont par exemple réalisés dans un matériau blindé contre les rayonnements ionisants tel que du plomb ou du tungstène. Le fond 12 ou la partie de guidage 11 sont par exemple réalisés dans un matériau blindé contre les rayonnements ionisants ou dans un autre matériau tel que de l'acier inoxydable.

Sur la figure 6, on voit que le poussoir biseauté 60 est fixé à un bouton 61 mobile dans un orifice 62 du fond 12. Les arceaux comprennent par ailleurs chacun une attache 58 longitudinale à un ressort 130 de rapprochement des arceaux, ces attaches 58 étant disposées à une extrémité à l'opposée de l'articulation 57 avec le fond. Le bouton 61 d'actionnement du poussoir biseauté 60 permet de faciliter le décrochage de la tête de piston 15, ce décrochage étant réalisé manuellement. Le poussoir biseauté 60 agit à l'opposé du ressort 130 de rapprochement des extrémités biseautées 59 arceaux 18.

Sur la figure 7, on voit que les arceaux 18 comprennent des logements 57 dans lesquels passent des axes 129 d'articulation fixés au fond 12. Les arceaux 18 comprennent chacun une extrémité biseautée 59, un poussoir biseauté 60 destiné à écarter les arceaux 18 étant par exemple disposé entre ces extrémités biseautées 59.

Ces arceaux 18 sont équipés chacun d'une oreille saillante 19 disposée vers l'intérieur du fourreau et destinée à venir contre une face de la tête 17 du piston 15 lorsque l'autre face de cette tête 17 est disposée contre le fond 12.

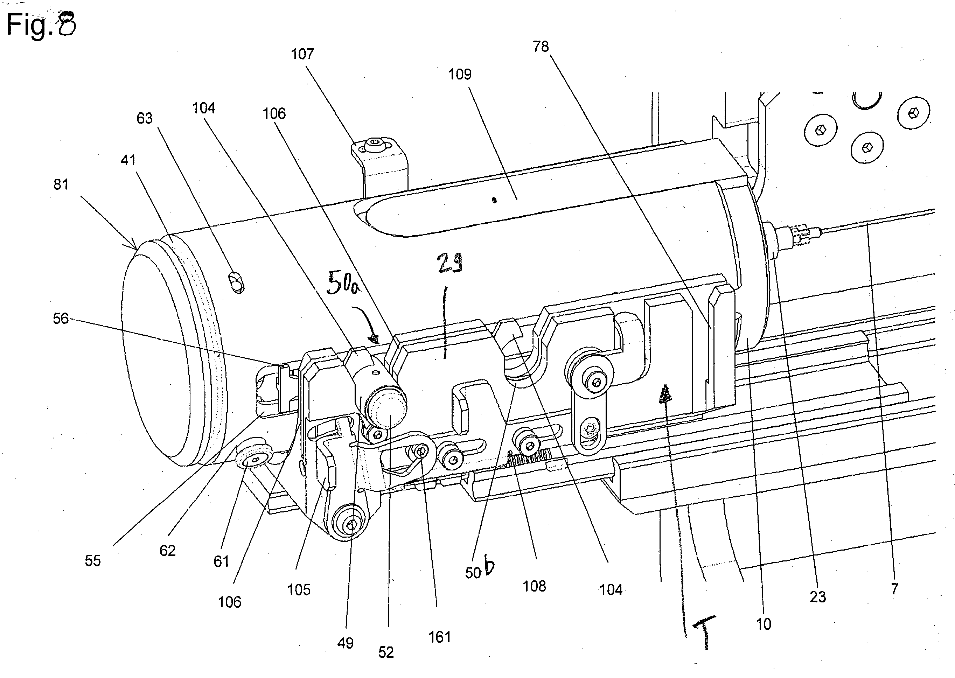

Sur la figure 8, on a représenté l'organe de transfert T et de maintien de la seringue S disposée dans le fourreau F. L'organe de transfert T est commandé en translation et en rotation par rapport au bâti 2. L'organe de transfert T occupe une position déterminée au poste de remplissage 8 et une position déterminée au poste d'enlèvement 9 de l'aiguille.

Cet organe de transfert constitue un réceptacle de réception du fourreau sur le bâti 2 du dispositif 1. Ce réceptacle se présente par exemple sous la forme d'un berceau 29, mais d'autres formes pourraient être envisagées. Bien entendu, le berceau 29 présente un rayon de courbure correspondant à un rayon de la partie de guidage 11.

Le berceau récepteur comprend une paire de logements 50a de réception des axes 49 de préhension du fourreau. Ces logements 50a sont réalisés dans des cloisons latérales du berceau et en vis à vis l'un de l'autre. Ces logements 50a sont équipés chacun d'une surface de guidage arrondie. Les cloisons latérales du berceau sont liées par une cloison en arrondie sur laquelle peut reposer le fourreau. Le fourreau est maintenu immobile dans le réceptacle, notamment en appui contre le berceau et les logements 50a pour les axes 49 de préhension.

Le berceau 29 comprend deux passages 106 longitudinaux réalisés dans les cloisons latérales, ces passages 106 accueillant une paire de pattes 104 plates biseautées fermant en partie les logements 50a de réception pour les axes 49 de préhension. Ces pattes 104, par exemple mobiles en rotation ou en translation, sont maintenues dans une position de fermeture par un ressort de rappel et écartées vers une position de dégagement par un levier 105 mobile en rotation par rapport au berceau 29. La partie biseautée des pattes 104 permet leur écartement automatique lorsque les axes 49 sont appuyés sur les pattes 104 vers les logements 50a. Les pattes 104 sont réalisées avec une position de fermeture déterminée, de façon à venir en appui sur les axes de préhension lorsque le fourreau est immobilisé par rapport au berceau.

Le berceau 29 comprend un bras mobile 107 lié aux pattes 104 de manière à ce que le bras 107 ait une position déterminée lorsque les pattes 104 sont dans leur position de fermeture, cette position déterminée étant indicative d'un verrouillage correct du fourreau dans le berceau récepteur.

Le berceau 29 comprend une paire de logements 50b supplémentaires de réception des axes 49 de préhension du fourreau. Ces logements 50b supplémentaires sont réalisés dans les cloisons latérales du berceau et sont par exemple équipés des pattes 104 plates biseautées supplémentaires mobiles dans les passages 106. Les pattes biseautées 104 supplémentaires sont entraînées de même par le levier 105 ou par le ressort de rappel, ces logements 50b supplémentaires étant disposés en vis à vis l'un de l'autre et étant équipés de surfaces de guidage arrondies.

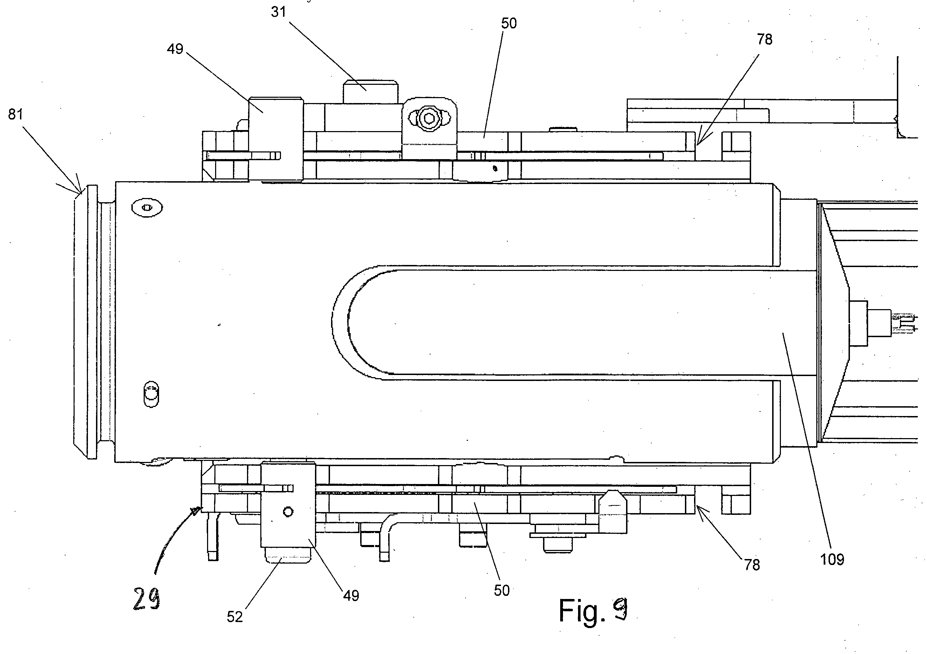

La figure 9 représente une vue de dessus du berceau 29 comprenant un deuxième doigt 31 disposé sur le côté dudit berceau et qui est destiné à venir en vis-à-vis du bâti 2. Ce doigt 31 est en saillie latéralement par rapport aux axes de préhension 49 maintenus dans le berceau 29.

Le berceau 29 comprend une rainure 78 aménagée de part et d'autre du berceau, la largeur de cette rainure 78, correspondant à une épaisseur d'une partie d'appui au poste de remplissage décrit ci-après. Cette rainure est disposée horizontalement lorsque le berceau 29 est disposé verticalement.

L'organe de transfert T permet d'amener la seringue S dans son fourreau F d'un poste à un autre poste et permet par exemple de positionner la seringue dans une position adéquate pour chacun de ces postes. Différentes postions de l'organe de transfert seront illustrées par la suite.

Le bouton 52 déporté à l'extrémité d'un des axes 49 permet à un opérateur d'accéder facilement à ce bouton, lorsque la seringue est disposée dans son fourreau qui est lui-même dans l'enceinte blindée 3. L'opérateur peut par exemple dégager la collerette 22 du crochet 51 et ainsi retirer le fond 12 encore attaché à la seringue, celle-ci se trouvant par ailleurs libérée du blindage tubulaire 10.

L'opérateur peut manipuler plus facilement la seringue allégée, pour disposer la seringue dans un puits 103 de mesure de l'activité radioactive. La seringue attachée au fond 12 et dégagée du blindage tubulaire 10, est vide ou remplie de fluide radioactif. De manière non limitative, la seringue attachée au fond 12 et dégagée du blindage tubulaire 10, est équipée de son aiguille 7 ou séparée de son aiguille 7.

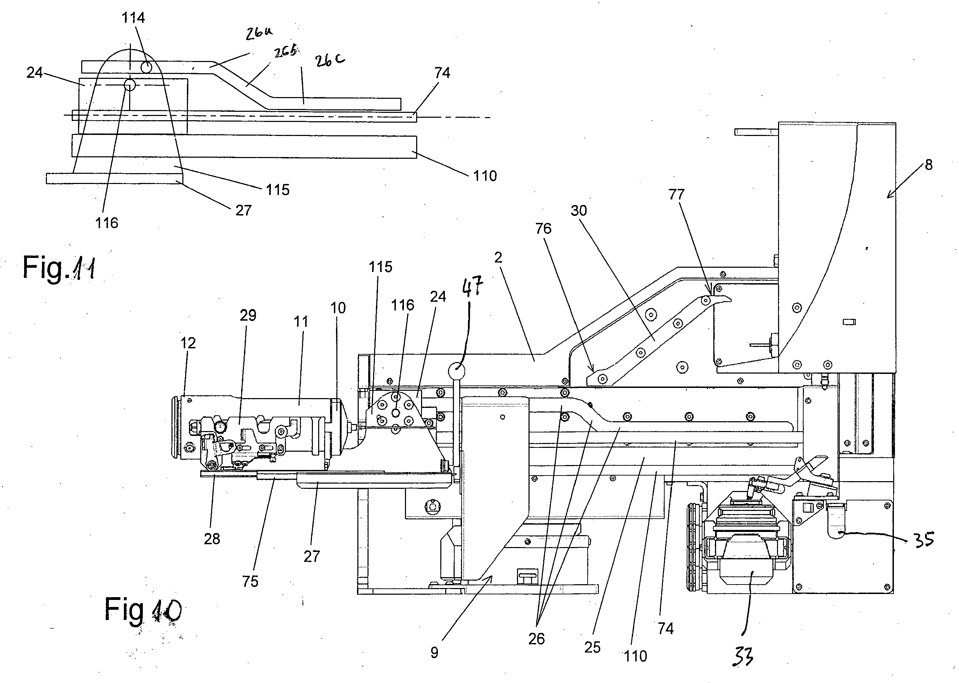

Sur la figure 10, l'organe de transfert comprend donc un module 24 mobile en translation dans une première rainure 25 rectiligne réalisée dans le bâti 2. Le module mobile en translation 24 est par exemple de forme parallélépipédique et comprend au moins une face en appui dans la première rainure 25. La face d'appui de la première rainure 25 est équipée d'une plaquette 110 fixée sur la face inférieure de la rainure 25 et venant en contact avec le module 24 mobile en translation. La première rainure est réalisée tout le long de la paroi du bâti 2. Cette première rainure est réalisée à la hauteur de l'ouverture 6 latérale de l'enceinte blindée 3. La première rainure va de l'ouverture 6 latérale au poste 8 de remplissage disposé par exemple à l'opposé de l'ouverture 6 latérale, en passant le long du poste 9 d'enlèvement.

Le module mobile 24 en translation comprend un trou fileté que traverse une tige filetée 74 commandée en rotation. La tige filetée 74 permet de commander la translation, dans un sens ou dans l'autre, du module mobile en translation 24. La tige filetée 74 est disposée tout le long de la première rainure 25, la tige 74 étant articulée aux deux extrémités de la première rainure 25.

La première rainure 25 est associée à une deuxième rainure 26 en forme de S, cette deuxième rainure commandant en hauteur un premier doigt 114 (visible sur la figure 11) guidé dans cette deuxième rainure 26. La deuxième rainure 26 est réalisée dans le bâti 2. La deuxième rainure 26 est réalisée dans le fond arrière de la première rainure 25.

Le premier doigt 114 a une section circulaire de diamètre correspondant à un écartement entre un bord supérieur et un bord inférieur de la deuxième rainure 26, de façon à guider le premier doigt 114 maintenu entre ces deux bords de la deuxième rainure 26. Le premier doigt est par exemple fixe par rapport à un premier plateau 27 de l'organe de transfert, le premier doigt et le premier plateau 27 étant mobiles en rotation par rapport au module mobile en translation 24.

Le premier plateau 27 supporte au moins le fourreau F et la seringue S. Le premier doigt 114 disposé horizontalement est fixé à la partie verticale 115 d'une pièce au profil en L, la partie horizontale du L formant par exemple le premier plateau 27. Une liaison pivotante 116 est réalisée entre le module mobile en translation 24 et la partie verticale 115 attachée au premier plateau 27. Cette partie en L est pivotante par rapport au module mobile en translation 24, selon un axe horizontal.

La deuxième rainure 26 comprend des parties rectilignes reliées entre elles par des coudes. La deuxième rainure 26 comprend une partie rectiligne horizontale allant de la porte 6 latérale de l'enceinte jusqu'au poste d'enlèvement 9, puis se poursuit après le poste 9 d'enlèvement par une partie rectiligne inclinée vers le bas en allant vers le poste 8 de remplissage. Cette partie rectiligne inclinée se poursuit par une partie rectiligne horizontale allant jusqu'au poste 8 de remplissage.

La deuxième rainure 26 disposée parallèlement à la première rainure 25 correspond à une translation de l'organe de transfert sans changement d'inclinaison.

La deuxième rainure 26 disposée inclinée par rapport à la première rainure 25 correspond à une translation de l'organe de transfert combinée à un changement d'inclinaison.

Un écart de hauteur déterminé entre deux portions horizontales de la deuxième rainure 26 correspond à un changement d'orientation d'un angle déterminé. De façon avantageuse, un positionnement de l'organe de transfert par rapport au bâti 2, correspond à une orientation déterminée en fonction du positionnement en translation du module mobile en translation 24.

L'organe de transfert T comprend un deuxième plateau 28 en liaison avec le premier plateau 27, le premier plateau 27 étant mobile en translation, selon une course déterminée, par rapport au deuxième plateau 28. Le fourreau F est fixé de façon amovible au berceau 29 fixé sur ce deuxième plateau 28.

Un plateau intermédiaire 75 est en contact d'une part avec le premier plateau 27 et d'autre part avec le deuxième plateau 28. Les plateaux 28, 29 ou 75 de l'organe de transfert sont mobiles en translation les uns par rapport aux autres grâce à leur forme formant des glissières. Les bords des plateaux sont d'une forme coopérant l'une avec l'autre de manière à réaliser un guidage en translation.

Cette liberté de mouvement supplémentaire permet par exemple d'augmenter les possibilités de positionnement de la seringue et de son fourreau, aux différents postes.

Une rampe 30 de commande en élévation est fixée au bâti 2. Le deuxième doigt 31 du berceau 29 vient en appui sur le dessus de la rampe 30 de commande en élévation par laquelle le berceau 29 et le deuxième plateau 28 peuvent être commandés en hauteur. La différence de hauteur entre une première extrémité de cette rampe 30 et une deuxième extrémité de cette rampe correspond par exemple à l'élévation du berceau 29 par rapport au premier plateau 27.

Une extrémité 76 de cette rampe 30 est disposée à la verticale d'une portion de la deuxième rainure 26 ayant varié en hauteur par rapport à la première rainure 25, de manière à accrocher le deuxième doigt 31 du berceau 29 redressé. Le berceau 29, s'accrochant par le deuxième doigt 31 à la rampe, est par exemple vertical ou redressé et formant un angle de quelques degrés par rapport à la verticale, comme représenté à la figure 9. La rampe 30 s'étend avantageusement jusqu'au poste de remplissage 8.

Sur la figure 11, on a représenté en détail la réalisation de la rainure 26 comprenant deux parties d'extrémité 26a et 26c décalées verticalement et reliées par une partie de jonction 26b. Cette partie 26b permet de faire passer le doigt 114 d'une position supérieure à une position inférieure.

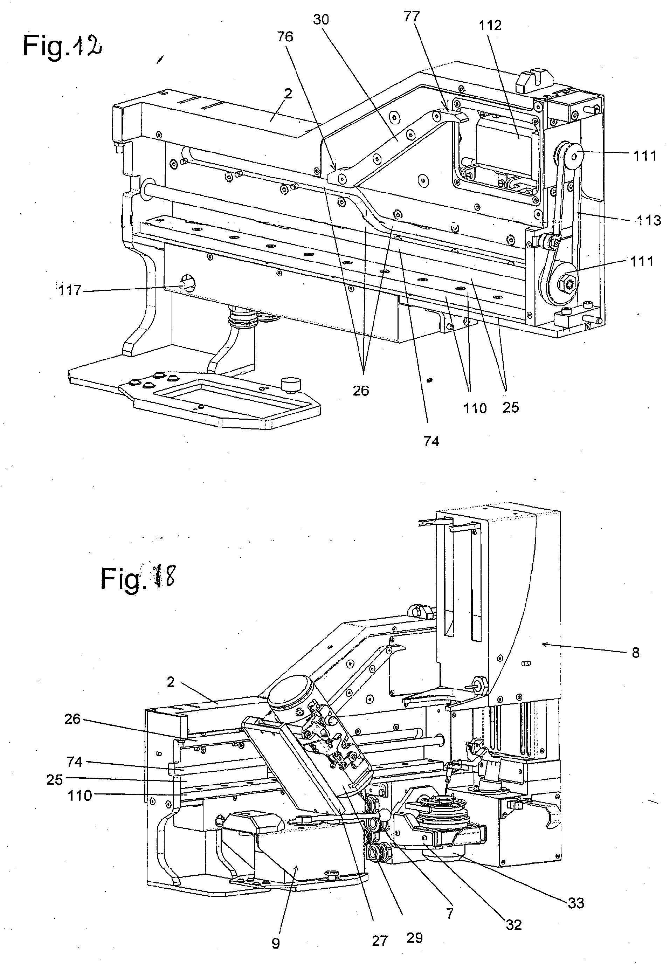

La figure 12 montre un moteur 112 intégré au bâti 2, entraînant par des poulies 111 et une courroie 113 la tige filetée 74. Un ou plusieurs capteurs, tel le capteur 117, sont par exemple montés sur le bâti 2 de manière à détecter la position du berceau 29 ou du module mobile en translation 24 ou d'autres éléments mobiles.

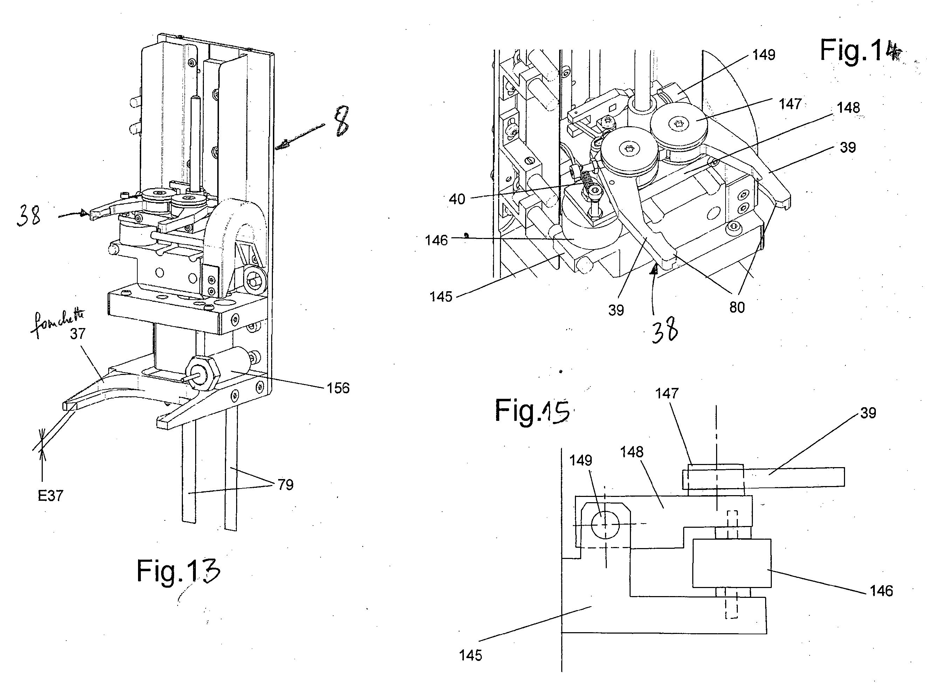

Sur les figures 13 à 15, on a représenté le poste de remplissage 8 de la seringue S permettant de prélever dans un flacon de stockage du liquide radioactif. Ce poste permet notamment l'abaissement du fourreau pour la perforation du flacon, la mobilité du piston 15 de la seringue.

Sur la figure 13, le poste 8 comprend une pince 38 et une fourchette 37 mobiles simultanément et séparément. La pince est destinée à être abaissée et clippée dans la rainure 41 du fourreau F. L'accrochage par la pince 38 permet le déblocage en translation du fond du fourreau par rapport au berceau 29, par actionnement du poussoir 156. La masse du fond 12 a en effet tendance à entraîner un enfoncement du piston dans le réservoir de la seringue.

La pince 38 est ensuite immobilisée par rapport à la fourchette 37 lors d'une descente ou d'une montée de cette fourchette 37. Un poussoir 156 peut aussi être commandé pour bloquer ou débloquer la translation du fond 12 du fourreau par rapport au berceau 29.

La descente de la fourchette 37 permet d'introduire l'aiguille 7 dans le flacon 33, comme cela sera décrit en relation avec la figure 18. La remontée de la fourchette 37 permet de sortir l'aiguille du flacon 33 et de positionner celle-ci au-dessus du flacon, le deuxième doigt 31, dans une position relevée étant disposée juste au-dessus de la deuxième extrémité 77 de la rampe 30.

Une translation de l'organe de transfert en position relevée permet de positionner le deuxième doigt 31 contre le dessus de la rampe 30 tout en dégageant le berceau 29 de la fourchette 37 et en dégageant la rainure 41 de la pince 38.

Lorsque la fourchette 37 est ramenée en position haute, le fond du fourreau étant bloqué en translation, la pince 38 est rapprochée de la fourchette 37, de façon à ne plus exercer d'effort dans la rainure 41. Le module 120 électronique de gestion stoppe la translation de la pince 38, lorsque le niveau d'effort est inférieur à un seuil déterminé, la pince 38 n'exerçant plus l'effort en translation sur le fond 12.

Le fond 12 du fourreau est bloqué en translation avant et après le remplissage en produit radioactif. Le fond du fourreau est débloqué en translation pendant la phase de remplissage, le fond étant alors porté par la pince 38.

En position baissée de la fourchette, une translation de la pince 38 en s'éloignant de la fourchette 37 permet d'entraîner le piston 15 dans le réservoir 21 de la seringue de façon à créer une aspiration par l'aiguille 7 et de façon à aspirer du fluide radioactif dans le réservoir 21. L'inclinaison du flacon 33 de réserve de produit radioactif va être décrite par la suite.

Le module électronique de gestion analyse par exemple le signal du capteur 146 afin de détecter une augmentation de l'effort de traction représentative du début d'aspiration du produit. L'éloignement du piston est commandé selon une course déterminée à partir du début de l'aspiration.

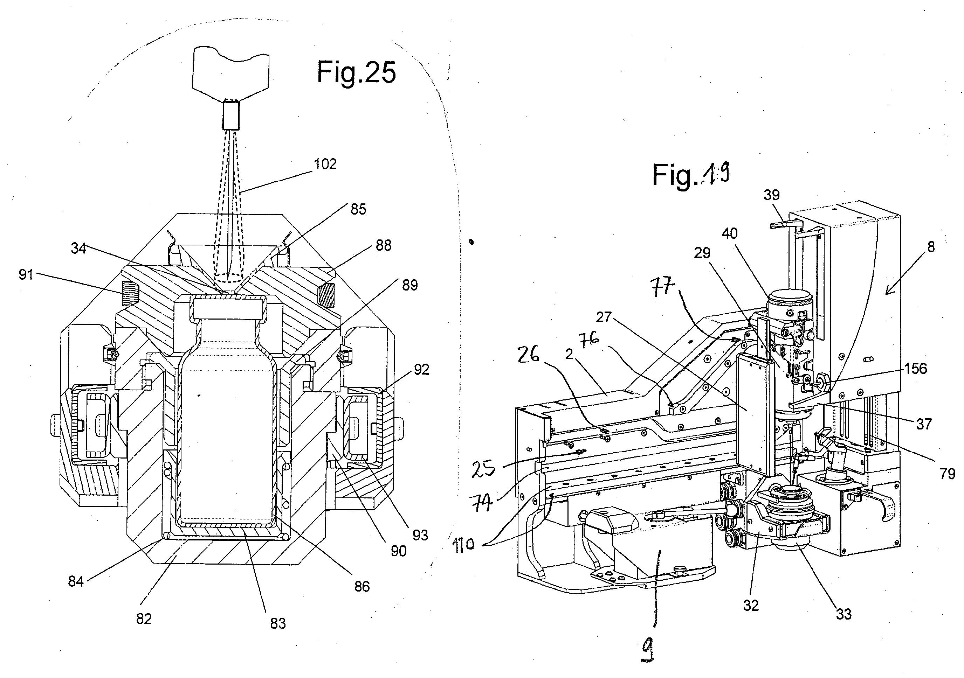

Les figures 19, 20 et 21 montrent de manière non limitative, le dispositif de transfert positionné au poste de remplissage, son premier plateau 27 gardant par exemple la même position par rapport au bâti 2. Un positionnement relatif des plateaux de l'organe de transfert, permet d'avoir plusieurs positions du fourreau au poste de remplissage.

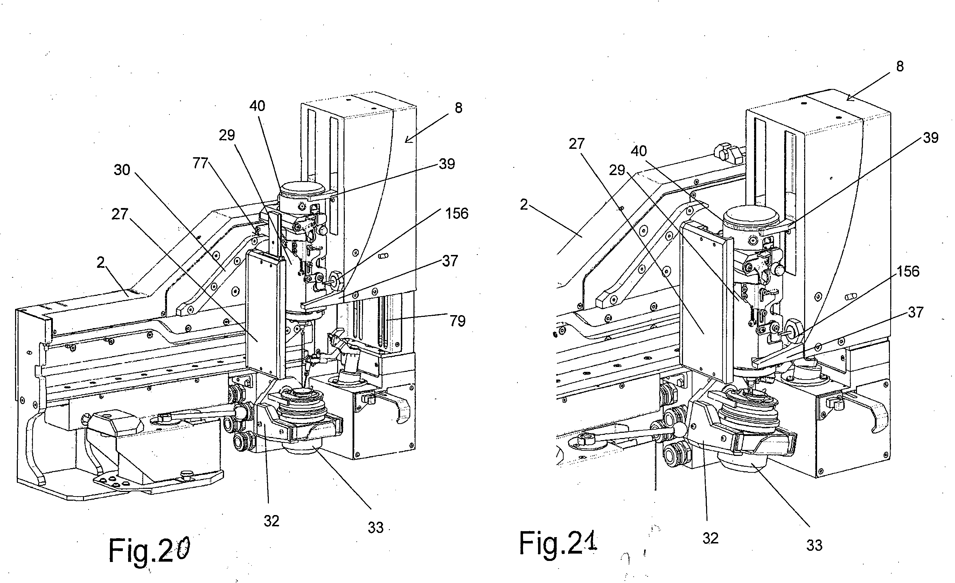

La seringue est disposée horizontalement avec son aiguille 7 comme représenté sur la figure 10 et finalement pointée vers le bas dans la position de remplissage comme représenté sur la figure 20 ou 21. Mais la seringue au poste de remplissage peut être verticale ou inclinée de quelques degrés par rapport à la verticale. L'élévation du berceau grâce à la rampe 30, permet par exemple d'amener l'aiguille 7 au-dessus du flacon.

Différentes configurations du poste de remplissage 8 vont maintenant être décrites.

Sur la figure 18, on a représenté le berceau 29 en position inclinée le doigt 114 ayant parcouru la première portion 26a de la rainure 26.

La figure 19 représente le berceau 29 positionné en position verticale dans la fourchette 37 qui est dans la position d'attente du berceau 29 et est accrochée à celui-ci. Dans cette position, le berceau 29 a parcouru complètement la rainure 26.

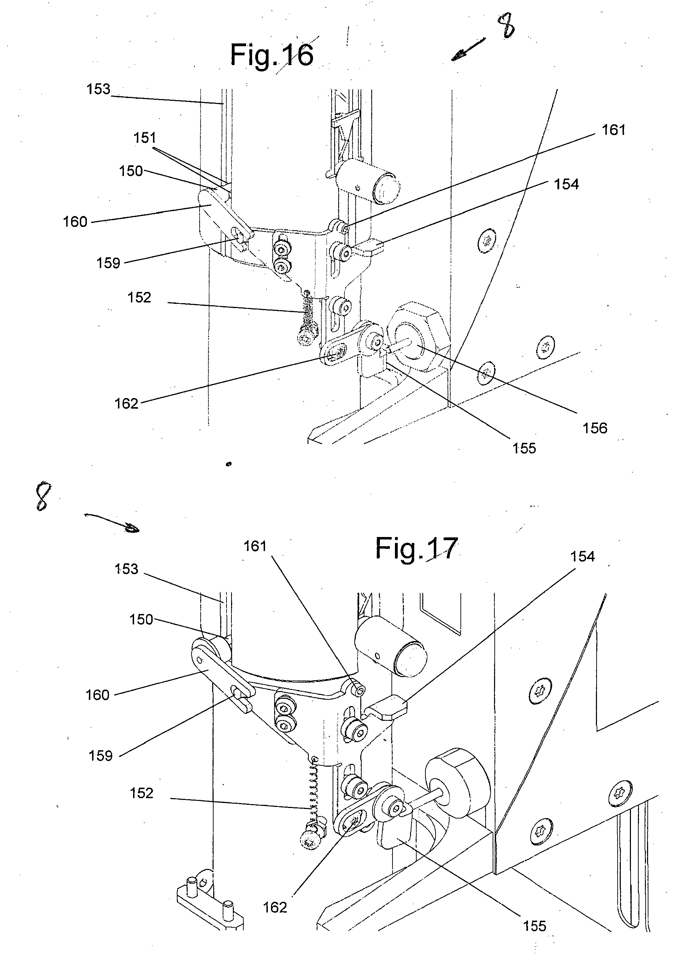

Comme représenté aux figures 16 et 17, le réceptacle comprend un élément de blocage de la partie 11 de guidage activé ou désactivé notamment au niveau du poste 8. Pour plus de clarté, les cloisons latérales et la cloison de fond du berceau 29 ne sont pas représentées aux figures 16 et 17. L'élément de blocage est ici réalisé sous la forme d'un doigt 150 monté pivotant dans le réceptacle et équipé, par exemple, de deux méplats 151 de réduction de sa largeur. Ce doigt coopère avec une fente 153 de blocage réalisée dans la partie de guidage 11.

L'élément de blocage est entraîné par un élément de rappel 152, tel qu'un ressort, tendant à entraîner l'élément de blocage dans une position de blocage en translation du fond 12 par rapport au berceau 29.

L'élément de blocage est entraîné d'autre part par un ou plusieurs éléments d'entraînement, référencés 154 ou 155, dans une position de libre translation du fond 12 par rapport au berceau 29.

Le doigt 150 est commandé en rotation par un des éléments d'entraînement de façon à se positionner avec ses méplats à distance de la fente dans la position de libre translation, comme représenté à la figure 17, les méplats étant alors parallèles aux bords de la fente 153 de blocage. Les méplats 151 sont en appui contre les bords de la fente de blocage dans la position de blocage en translation. Le réceptacle comprend deux éléments d'entraînement du doigt de blocage dans la position de libre translation, dont un élément d'entraînement 154 actionné manuellement et un élément d'entraînement 155 actionné par un poussoir.

L'élément d'entraînement 154 peut par exemple être actionné à la main selon un mouvement de translation. Cet élément d'entraînement 154 comprend des trous oblongs traversés par des tiges de vis, limitant sa course en translation. Le ressort 152 tend à entraîner l'élément 154 en butée de course, cet élément 154 comprenant un plot 159 d'entraînement d'une fourche 160 fixée à l'extrémité du doigt 150.

Le doigt 150 est mobile en rotation dans une cloison du réceptacle. Le doigt 150 comprend une partie équipée de deux méplats 151 saillante à l'intérieur du berceau 29.

L'élément 154 comprend un plot 161 par lequel il est entraîné par le levier 105. Le levier 105 comprend un évidement 163 coopérant avec le plot 161. Le levier 105 entraîne l'élément 154 d'entraînement vers la position de libre translation, tandis que l'évidement 163 est suffisamment étendu pour permettre un libre déplacement du plot 161 qui n'entraîne pas réciproquement le levier 105.

L'élément d'entraînement 154 est d'autre part susceptible d'être entraîné directement par une partie de préhension recourbée vers l'extérieur du réceptacle et accessible manuellement.

L'élément d'entraînement 154 est d'autre part susceptible d'être entraîné par un élément 155 d'entraînement par poussoir présentant une partie de préhension recourbée vers l'intérieur du réceptacle. Cette partie recourbée vers l'intérieur du réceptacle est actionnée par un poussoir piloté automatiquement. Un plot 162 permet par exemple l'entraînement réciproque de l'élément d'entraînement 154 et de l'élément d'entraînement 155. L'élément d'entraînement 155 a une forme en L dont une branche comprend un trou oblong de coopération avec le plot 162 et l'autre branche comprend la partie recourbée vers l'intérieur du réceptacle. L'élément d'entraînement 155 en forme de L est par exemple monté pivotant sur le berceau.

La phase de remplissage va maintenant être décrite en référence aux figures 13 à 15.

Comme décrit précédemment, le poste de remplissage 8 comprend par exemple une fourchette 37 de support du berceau 29. La fourchette 37 est mobile verticalement. La fourchette 37 est commandée en translation de façon automatique. La fourchette 37 est guidée sur des glissières verticales 79.

La fourchette 37 présente ici une forme de U dont les extrémités des pattes sont disposées vers le berceau arrivant par la rampe 30. La fourchette 37 comprend une surface d'appui plate équipée de rebords latéraux entourant en partie le berceau. La surface d'appui plate de la fourchette est horizontale. Le berceau 29 arrive entre les deux pattes de la fourchette 37 qui est disposée à une hauteur déterminée correspondant à la hauteur des surfaces 78 d'appui du berceau 29. La fourchette 37 s'insère dans la rainure 78 du fourreau.

La pince 38 comprend des branches 39 rapprochées automatiquement l'une vers l'autre par un élément de rappel 40, tel qu'un ressort. Les branches 39 s'accrochent automatiquement dans la rainure 41 du fourreau et de manière à pouvoir exercer un effort en translation verticale.

Les extrémités 80 des branches 39 épousent la forme intérieure de la rainure 41. Ces extrémités 80 sont prolongées par des parties formant un élargissement de manière à s'écarter plus facilement au contact du bord 81 biseauté du fourreau.

Selon une variante de réalisation non limitative, on pourrait aussi avoir une pince 38 dont les branches sont commandées en rapprochement ou en écartement par un actionneur piloté par le module 120 électronique de gestion.

Les branches 39 sont montées sur un support 145, ce support étant commandé en translation par rapport à la fourchette 37. Le support 145 est équipé d'un capteur 146 qui produit des données représentatives de l'effort en translation verticale. Ces données sont transmises, par une liaison électrique, au module électronique de gestion.

Le capteur 146 de mesure de l'effort en translation verticale, est monté en appui sur le support 145 et disposé sous des articulations 147 des branches 39. Les branches 39 de la pince 38, sont articulées avec un bras 148 lui-même articulé avec le support 145, le capteur 146 supportant par exemple la masse des branches 39. Le capteur 146 est disposé en liaison avec une extrémité du bras 148, cette extrémité étant à l'opposé de l'articulation 149 du bras avec le support 145 des branches.

La pince 38 est commandée en translation de façon à se rapprocher ou à s'éloigner de la fourchette 37, par un passage fileté réalisé dans le support 145 dans lequel passe une tige filetée commandée en rotation dans un sens ou dans l'autre.

Comme représenté à la figure 16, un poussoir 156 du poste de remplissage peut agir sur l'élément 155 d'entraînement du doigt 150 de blocage. Le poussoir est déplacé sous l'action d'un actionneur électromagnétique déplaçant une tige de poussoir 156 dans un sens ou dans l'autre. Le poussoir 156 est disposé en face de l'élément d'entraînement 155 du doigt 150 de blocage lorsque le berceau 29 est en place dans la fourchette 37. Le fourreau peut ainsi être maintenu bloqué ou avec son fond 12 libre en translation, au poste 8 de remplissage.

Le processus de préparation de la seringue va maintenant être décrit depuis sa position initiale de la figure 8 jusqu'à sa position finale par translation du fourreau et du berceau 29.

Le fourreau est tout d'abord disposé dans le berceau 29, lors de l'étape de chargement du fourreau dans le réceptacle. Le chargement est par exemple réalisé manuellement par l'opérateur.

Lorsque la fermeture de toutes les portes est détectée, l'étape suivante de remplissage de la seringue est exécutée.

A la fin du remplissage, le transfert du fourreau à un poste d'enlèvement de l'aiguille est exécuté.

Après la détection du poste d'enlèvement de l'aiguille disposé en position active, on procède à l'enlèvement de l'aiguille.

Après l'enlèvement de l'aiguille et son stockage dans un coffre blindé, le transfert du fourreau au poste de déchargement est commandé.

Le fourreau positionné au poste de chargement/déchargement est au moins en partie à l'extérieur de l'enceinte blindée 3. Le déchargement est réalisé manuellement par l'opérateur.

Les différentes étapes de transfert de l'organe de maintien et de transfert vont maintenant être décrites en relation avec les différents postes du dispositif de préparation de la seringue et les figures 18 à 21.

Comme cela a été décrit en relation avec les figures 1 et 2, une extension des plateaux permet de sortir le fourreau à l'extérieur de l'enceinte blindée 3. La sortie du fourreau à l'extérieur de l'enceinte 3 permet de faciliter les manoeuvres, pour positionner le fourreau dans le berceau 29 ou pour retirer le fourreau du berceau 29. Les matériaux isolant des rayonnements ionisants, tel le plomb ou le tungstène, sont en effet utilisés avec des épaisseurs importantes pour réaliser le fourreau, le fourreau pouvant alors peser plusieurs kilogrammes et étant difficile à manipuler.

Sur la figure 18, on voit que le berceau 29, entraîné par le module 24 et le doigt 114, occupe une position inclinée par passage des doigts 114 et 31 dans la rainure 26 et sur la rampe 30 et par coulissement du plateau 28 par rapport au plateau 27.

Sur la figure 19, on voit que le fourreau et la seringue sont en position verticale pris en charge par la fourchette 37 et les pinces 38.

Sur la figure 20, on voit que les branches 39 ont été abaissées pour venir se positionner dans la rainure 41 du fourreau.

Sur la figure 21, le fourreau pris en charge à la fois par les pinces 38 et branches solidaires en translation est abaissé pour venir perforer le bouchon du réservoir 33 afin de procéder au prélèvement de la dose de produit radioactif nécessaire au patient. On commande alors la translation des branches 39 qui entraînent alors la base 81 du fourreau et conséquemment le piston 15 pour remplir la seringue.

Le fourreau et la seringue sont ensuite ramenés en position initiale en passant par une phase intermédiaire d'enlèvement de l'aiguille 7.

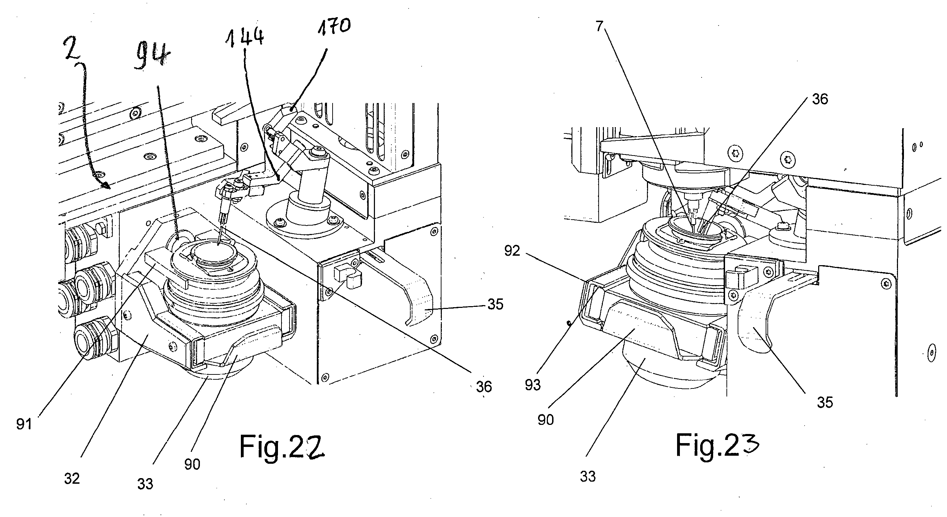

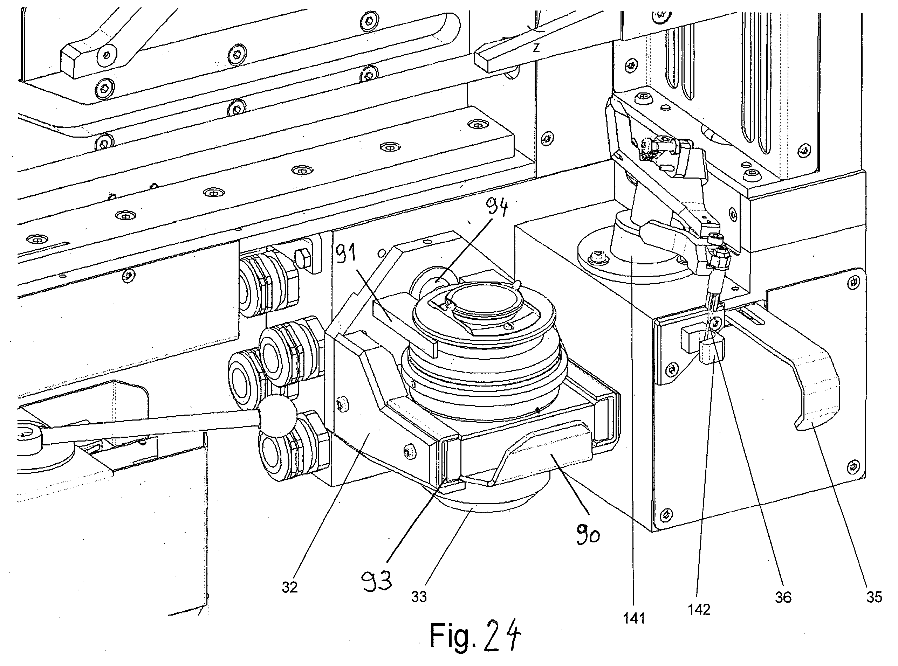

Le poste de stockage du produit de réserve va maintenant être décrit en référence aux figures 22 à 25.

Sur les figures 22 et 23, le flacon 33 de réserve de produit radioactif est maintenu dans une pièce périphérique 90 comprenant des glissières latérales 93. Ces glissières latérales 93 viennent coulisser dans un logement 92 de l'organe 32 de maintien, permettant ainsi de le translater à la manière d'un tiroir. La pièce 90 entourant le flacon comprend par exemple une poignée de préhension.

L'organe 32 de maintien comprend une fourchette 91 de positionnement du flacon 33. Cette fourchette 91 vient s'accrocher dans une rainure périphérique du chapeau blindé 88. L'organe 32 de maintien comprend un axe 94 de rotation par rapport au bâti 2. L'organe de maintien 32 est positionné suivant l'axe central du flacon vertical ou incliné de quelques degrés, par un organe de commande d'inclinaison.

L'organe de commande d'inclinaison comprend un ensemble mécanique à tringles et pivots grâce auquel la descente du bloc comprenant la fourchette, entraîne automatiquement la rotation de l'organe 32 de maintien.

Selon un exemple de réalisation, l'organe de commande d'inclinaison est par exemple piloté par le module électronique de gestion, de façon automatique en fonction de la position du poste de remplissage.

Comme représenté à la figure 25, le flacon 33 est fermé par un couvercle transperçable 34. Le flacon 33 est constitué d'un conteneur blindé équipé d'un guide conique 85 fermé par le couvercle 34 transperçable et d'un fond blindé 82 formant un logement interne recevant une douille 83 interne.

La douille interne 83 est poussée par un ressort 84 en appui d'autre part contre l'intérieur du fond 82 blindé. Le réceptacle 83 comprend une fiole 86 de fluide radioactif et/ou un adaptateur de taille, non représenté, dans ou sur lequel est disposée la fiole 86.

Le conteneur 33 comprend un chapeau blindé 88 recevant le cône 85 de guidage fermé par le couvercle 34 transperçable. Le conteneur 33 comprend enfin un manchon blindé 89 s'imbriquant avec le fond blindé 82 et avec le chapeau 88 blindé.

Une position droite de l'organe de maintien est représentée à la figure 22 et une position inclinée est représentée à la figure 23. La position inclinée du flacon 33 permet à l'aiguille 7 de la seringue d'occuper différentes positions relatives par rapport au flacon. Le flacon 33 est par exemple positionné droit avant la pénétration de l'aiguille 7. L'aiguille 7 perfore ensuite le couvercle 34, par une descente de la fourchette 37, comme représenté à la figure 21. L'axe du cône 85 de guidage est aligné avec l'axe longitudinal de la seringue, pour une meilleure pénétration de l'aiguille qui ne risque pas de percuter le bord intérieur latéral du flacon. Le module électronique de gestion ou un ensemble mécanique commande ensuite automatiquement l'inclinaison du flacon 33. L'aiguille 7 descend d'une hauteur déterminée de façon à ce que la pointe de l'aiguille 7 arrive dans la partie inférieure du flacon 33. L'aspiration de la dose nécessaire est alors réalisée.

Le système peut nécessiter une aiguille d'appel d'air 36 qui va maintenant être décrite en relation avec les mêmes figures. Cette aiguille d'appel d'air 36 est en liaison avec le bâti 2. Cette aiguille d'appel d'air 36, distincte de l'aiguille 7 de la seringue, a une inclinaison correspondant à la position inclinée du flacon 33. L'aiguille d'appel d'air 36 est mobile par rapport au bâti 2 et commandée en translation selon son axe central. De façon avantageuse, l'axe du cône 85 de guidage, en position incliné de celui-ci, est aligné avec l'axe de l'aiguille d'appel d'air, pour une meilleure pénétration de l'aiguille d'appel d'air qui ne risque pas elle non plus de percuter le bord intérieur latéral du flacon 33.

L'aiguille 36 d'appel d'air peut être commandée en translation par un ensemble mécanique à tringles et de pivots grâce auxquels la descente du bloc comprenant la fourchette, entraîne la translation de cette aiguille.

Selon un exemple de réalisation, l'aiguille d'appel d'air 36 est par exemple pilotée, par le module électronique de gestion, de façon automatique en fonction de la position du poste de remplissage.

L'aiguille d'appel d'air 36 est utilisée lors du remplissage de la seringue. Le flacon 33 est positionné sensiblement verticalement avant la pénétration de l'aiguille 7, comme représenté à la figure 20. L'aiguille 7 perfore ensuite le couvercle 34, par une descente de la fourchette 37, comme représenté à la figure 21. L'inclinaison du flacon 33 et l'avancée de l'aiguille 36 d'appel d'air sont par exemple ensuite commandées.

L'aiguille 7 de la seringue descend par exemple d'une hauteur déterminée de façon à ce que la pointe de l'aiguille 7 arrive dans la partie inférieure du flacon 33. Une aspiration est ensuite réalisée.

Il va de soi que l'aiguille 36 d'appel d'air permet de réaliser une arrivée d'air à l'intérieur du flacon 33 et permet de faciliter l'aspiration du fluide.

L'opérateur peut dégager l'aiguille d'appel d'air 36. Une manette 35 actionnée manuellement agit sur un support 141 de l'aiguille d'appel d'air 36 de façon à positionner le support 141 de l'aiguille d'appel d'air 36 dans au moins une position fonctionnelle ou une position de dégagement.

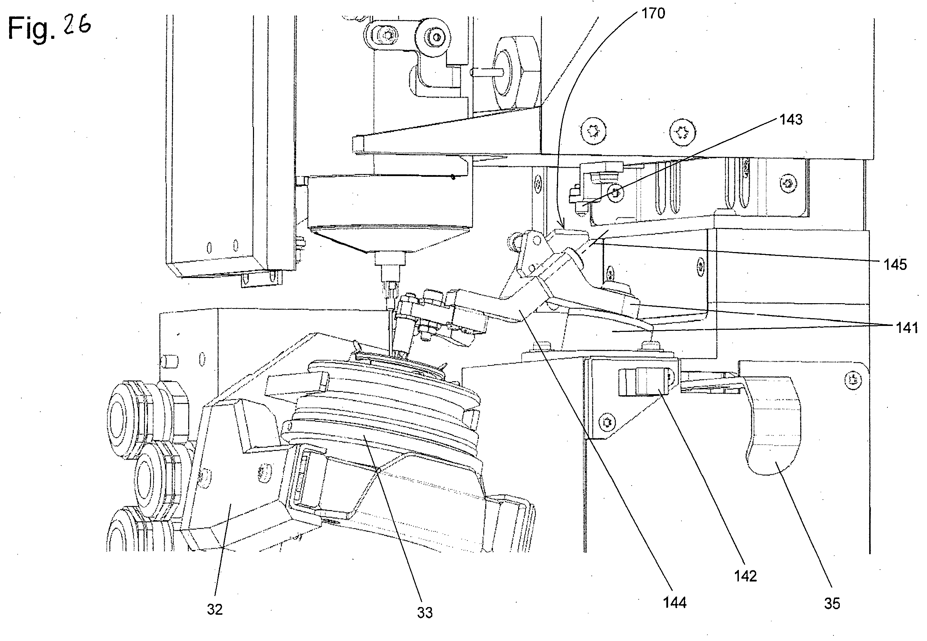

Sur la figure 26, l'aiguille d'appel d'air 36 dans la position de dégagement reste par exemple commandée en translation à l'intérieur d'une cloison de protection 142. La cloison de protection a la forme d'un U ouvert sur un côté latéral. Lors d'un pivotement de dégagement du support 141, l'aiguille 36 passe par cette ouverture latérale. La manette 35 mobile en translation est équipée d'un ensemble de tringles ou d'une crémaillère agissant sur des dents du support 141 mobile en rotation par rapport au bâti 2. Des capteurs contrôlant la position angulaire du support 141, sont en communication avec le module électronique de gestion, de façon à transmettre des signaux représentatifs de la position de l'aiguille d'appel d'air 36.

Le support 141 de l'aiguille d'appel d'air 36 porte un bras 144 descendant vers l'organe de maintien du flacon 33. Ce bras 144, auquel est rattachée l'aiguille d'appel d'air 36, est monté pivotant par rapport au support 141 de façon à réaliser un décalage lors d'une descente de l'aiguille d'appel d'air 36.

La partie du poste de remplissage 8, fixée à la fourchette 37 et mobile en translation, comprend un poussoir 143 destiné à agir sur le bras 144, comme représenté à la figure 26. Le poussoir 143 comprend une bille bloquée dans un logement à son extrémité et destinée à venir contre le bras 144. Le bras 144 comprend une partie 170 décalée par rapport à son axe 145 de rotation et placée sous le poussoir à bille 143, destinée à venir contre le poussoir 143. Cette partie 170 décalée permet par exemple d'entraîner le bras 144 en rotation autour de son axe 145 de façon à réaliser un léger décalage, le poussoir à bille 143 n'agissant sur le bras 144 qu'en fin de course.

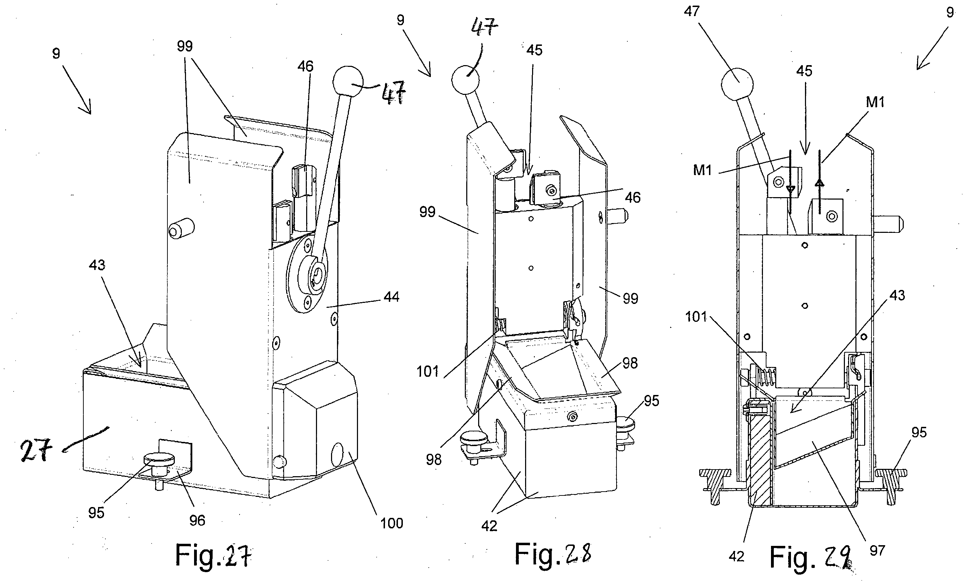

L'enlèvement de l'aiguille va maintenant être décrit en référence aux figures 27 à 30.

Le poste d'enlèvement 9 de l'aiguille 7 comprend un outil escamotable dans une position passive. L'outil escamotable est apte à occuper une position active dans laquelle un coffre de stockage 42 de l'aiguille 7 est ouvert automatiquement. Le coffre 42 peut comporter des bords blindés. Des pattes 96 sont fixées au coffre, ces pattes comprenant par exemple un passage de réception d'une vis 95 de fixation du coffre au bâti 2. Le coffre est fixé au bâti 2 par des vis serrées manuellement.

Le coffre comprend un double fond dont la paroi inférieure 97 est inclinée vers un coin du double fond, permettant avantageusement un calage d'une aiguille stockée. Le double fond comprend des parois latérales 98 se terminant par des bords inclinés permettant avantageusement de guider une aiguille 7 dans sa chute après son dévissage. Le double fond est simplement posé dans le coffre 42 et peut être retiré pour récupérer les aiguilles dévissées. Le coffre 42, en liaison avec le bâti 2, présente une ouverture 43 disposée sous l'aiguille 7 de la seringue au poste d'enlèvement.

L'outil escamotable comprend un panneau de fermeture 44 de l'ouverture du coffre 42. Le panneau 44 est positionné verticalement dans sa position active. Le panneau 44 est prolongé par des panneaux 99 disposés perpendiculairement venant de chaque côté du coffre dans la position escamotée. Le panneau 44 comprend un passage 45 ouvert jusqu'en haut du panneau et destiné à recevoir une base 48 dévissable de l'aiguille 7. L'outil escamotable comprend par exemple des patins 46 disposés de chaque côté du passage 45 et actionnés chacun, par un levier de commande 47, selon au moins un mouvement translatif M1 de dévissage en contact avec la base 48.

Le levier 47, monté pivotant par rapport au panneau 44, active par exemple un mécanisme de mise en mouvement des patins 46, ce mécanisme étant interne au panneau 44.

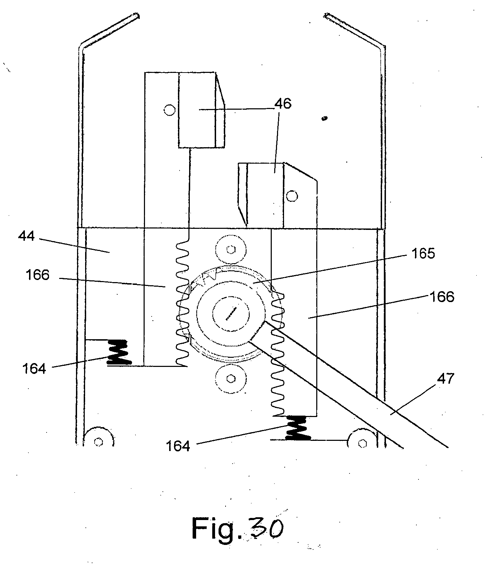

Comme représenté à la figure 30, les patins sont fixés chacun à une crémaillère 166 entraînée par un pignon 165 à dents entraîné en rotation par le levier 47. Les crémaillères 166 sont ramenées dans leur position d'attente par des ressorts 164 de rappel.

Un contrepoids 100 est fixé au panneau 44 et permet d'équilibrer le panneau 44 dans son mouvement de basculement. Le mouvement de basculement du panneau 44 peut être aidé par un ressort 101 disposé autour de son axe de rotation.

Il doit être évident pour l'homme du métier que la présente invention permet d'autres variantes de réalisation. Par conséquent, les présents modes de réalisation doivent être considérés comme illustrant l'invention définie par les revendications jointes.

## Revendications

1. Fourreau (F) de protection et de préhension d'une seringue (S) équipée d'une aiguille (7), fourreau (F) comprenant d'une part un blindage tubulaire (10) fixé autour d'un réservoir (21) de la seringue, et d'autre part un fond (12), **caractérisé en ce que** ledit fourreau (F) comprend deux tourillons (49) support fixes par rapport à un blindage tubulaire (10), lesdits tourillons (49) délimitant une surface cylindrique d'appui dans des logements (50) d'un berceau (29), le berceau (29) délimitant un réceptacle de réception dudit fourreau et un élément de blocage (150, 151) du fond (12) par rapport au berceau (29), un élément de rappel (152) tendant à entraîner l'élément de blocage (150, 151) dans une position de blocage en translation du fond (12) par rapport au berceau (29).

2. Fourreau (F) de protection et de préhension d'une seringue (S) selon la revendication 1, **caractérisé en ce qu'**il comprend une enveloppe de guidage (11) disposée autour du blindage tubulaire (10), ladite enveloppe (11) étant équipée de fentes (54) dans lesquelles circulent les tourillons (49), ladite enveloppe étant reliée au fond (12) équipé d'un moyen d'attache (18, 19, 20, 57, 58, 63) à la tête (17) du piston (15) de la seringue.

3. Fourreau (F) de protection et de préhension d'une seringue (S) selon la revendication 2, **caractérisé en ce qu'**il comprend un crochet (51) mobile en rotation par rapport au blindage tubulaire (10), le crochet (51) réalisant un accrochage automatique d'une collerette (22) du réservoir (21) de la seringue, un bouton poussoir (52) étant disposé à une extrémité libre d'un des deux tourillons (49), le bouton poussoir agissant sur le crochet (51), à l'opposé d'un élément de rappel (53), pour dégager la collerette (22) du crochet, le crochet (51) étant escamoté par rapport au fourreau (10, 11, 12) dans sa position d'accrochage.

4. Fourreau (F) de protection et de préhension d'une seringue (S) selon la revendication 3, **caractérisé en ce que** le moyen d'attache comprend deux arceaux (18) articulés et rapprochés automatiquement l'un vers l'autre par un élément de rappel (130), les arceaux (18) étant équipés chacun d'une oreille saillante (19) disposée vers l'intérieur du fourreau et destinée à venir contre une face de ladite tête (17).

5. Dispositif de transport, de préparation ou d'injection d'une dose de fluide radioactif à injecter à un patient comprenant un fourreau selon l'une des revendications 1 à 4.

## Patentansprüche

1. Hülse (F) zum Schutz und zur Handhabung einer Spritze (S), welche mit einer Nadel (7) ausgestattet ist, wobei die Hülse (F) einerseits eine rohrförmige Abschirmung (10), welche um den Tank (21) der Spritze herum befestigt ist, und andererseits einen Boden (12) aufweist, **dadurch gekennzeichnet, dass** die genannte Hülse (F) zwei in Bezug auf eine rohrförmige Abschirmung (10) feste Stützzapfen (49) aufweist, wobei die genannten Zapfen (49) in Aufnahmen (50) eines Gestells (29) eine zylindrische Anschlagfläche abgrenzen, wobei das Gestell (29) einen Behälter zur Aufnahme der genannten Hülse und ein Element zum Blockieren (150, 151) des Bodens (12) in Bezug auf das Gestell (29) abgrenzt, wobei ein Rückholelement (152) bestrebt ist, das Blockierungselement (150, 151) in eine Position zur Blockierung des Bodens (12) in Verschieberichtung in Bezug auf das Gestell (29) zu bewegen.

2. Hülse (F) zum Schutz und zur Handhabung einer Spritze (S) nach Anspruch 1, **dadurch gekennzeichnet, dass** sie eine Führungshülle (11) aufweist, welche um die rohrförmige Abschirmung (10) herum angeordnet ist, wobei die genannte Hülle (11) mit Schlitzen (54) versehen ist, in welchen sich die Zapfen (49) bewegen, wobei die genannte Hülle mit dem Boden (12) verbunden ist, welcher mit einem Mittel zur Befestigung (18, 19, 20, 57, 58, 63) an dem Kopf (17) des Kolbens (15) der Spritze versehen ist.

3. Hülse (F) zum Schutz und zur Handhabung einer Spritze (S) nach Anspruch 2, **dadurch gekennzeichnet, dass** sie einen in Bezug auf die rohrförmige Abschirmung (10) drehbeweglichen Haken (51) aufweist, wobei der Haken (51) eine automatische Verrastung eines Bundes (22) des Tanks (21) der Spritze verwirklicht, wobei ein Druckknopf (52) an einem freien Ende eines der beiden Zapfen (49) angeordnet ist, wobei der Druckknopf auf den Haken (51) auf einer einem Rückholelement (53) gegenüberliegenden Seite einwirkt, um den Bund (22) mit dem Haken außer Eingriff zu bringen, wobei der Haken (51) in Bezug auf die Hülse (10, 11, 12) in seine Rastposition weggeschwenkt wird.

4. Hülse (F) zum Schutz und zur Handhabung einer Spritze (S) nach Anspruch 3, **dadurch gekennzeichnet, dass** das Befestigungsmittel zwei Bügel (18) aufweist, welche angelenkt sind und durch ein Rückholelement (130) automatisch zueinander geführt werden, wobei die Bügel (18) jeweils mit einem vorspringenden Lappen (19) versehen sind, welcher in Richtung des Inneren der Hülse angeordnet ist und dafür vorgesehen ist, mit einer Fläche des genannten Kopfes (17) in Eingriff zu gelangen.

5. Vorrichtung zum Transport, zur Vorbereitung oder zur Injektion einer Dosis von radioaktiver, einem Patienten zu injizierender Flüssigkeit, aufweisend eine Hülse nach einem der Ansprüche 1 bis 4.

## Claims

1. Protective grip sleeve (F) for a syringe (S) equipped with a needle (7), sleeve (F) comprising firstly a shielding tube (10) fitted around a reservoir (21) of the syringe, and secondly a base (12), **characterised in that** said sleeve (F) comprises two trunnion supports (49) fixed with respect to a shielding tube (10), said trunnions (49) delimiting a cylindrical bearing surface in the housings (50) of a cradle (29), the cradle (29) delimiting a reception receptacle for said sleeve and a blocking element (150, 151) for the base (12) with respect to the cradle (29), a return element (152) tending to drive the blocking element (150, 151) into a position in which it blocks the translation of the base (12) with respect to the cradle (29).

2. Protective grip sleeve (F) for a syringe (S) according to Claim 1, **characterised in that** it comprises a guiding envelope (11) arranged around the shielding tube (10), said envelope (11) being provided with slots (54) in which the trunnions (49) move, said envelope being linked to the base (12) equipped with attachment means (18, 19, 20, 57, 58, 63) to the head (17) of the syringe plunger (15).

3. Protective grip sleeve (F) for a syringe (S) according to Claim 2, **characterised in that** it comprises a hook (51) able to rotate with respect to the shielding tube (10), the hook (51) automatically hooking onto a collar (22) of the syringe reservoir (21), a push button (52) being arranged at a free end of one of the two trunnions (49), the push button acting on the hook (51), opposing a return element (53), to disengage the collar (22) from the hook, the hook (51) being retracted with respect to the sleeve (10, 11, 12) in its hooking position.

4. Protective grip sleeve (F) for a syringe (S) according to Claim 3, **characterised in that** the attachment means comprise two hinged hoops (18) automatically brought together by a return element (130), the hoops (18) each being equipped with a protruding lug (19) arranged towards the inside of the sleeve and intended to be positioned against one face of said head (17).

5. Device to carry, prepare or inject a dose of radioactive fluid to be injected into a patient and comprising a sleeve according to one of Claims 1 to 4.
